# EUROPEAN PATENT APPLICATION

(11) **EP 1 269 918 A1**
(43) Date of publication of application: **02.01.2003**
(21) Application number: 02013845.9
(22) Date of filing: 22.06.2002
(51) Int. Cl.: A61B 10/00

(54) **Endogastric capsule**

(30) Priority: 26.06.2001 IT PS20010019
(71) Applicant: Muretto, Pietro Aurelio Gaetano, 61100 Pesaro (IT)
(72) Inventor: Muretto, Pietro Aurelio Gaetano, 61100 Pesaro (IT)
(74) Representative: Gustorf, Gerhard, Dipl.-Ing.

(57) **Abstract**

Endogastric capsule (1) for taking samples of gastric juices comprising a removable absorbing medium (11) retained within a container (2) and communicating outside through at least one opening (10) as well as enclosed inside a dissolvable casing (3) and fastened to a string (4) for endogastric placement and relevant recovery.

## Description

### DETAILED DESCRIPTION

### State of the art

Most recent medical research has shown that the extensive presence of certain substances, known as tumorous *markers,* in the gastric juices can be related to stomach cancers, just as such foretelling high values are all the more ascribable to the finding of antigens related to carcinogenesis such as the so-called "CEA", acronym of carcinoembryonic antigen and "CA19.9", meaning carbohydrate antigen 19.9.

In view of this picture, it appears pretty obvious just how important the testing of gastric juices can be for finding such substances in significant concentrations for early diagnosis and therefore for an appropriate therapy for this kind of cancer which has one of the highest mortality rates.

### Limits of state of the art

The means for sampling gastric juices according to the state of the art are rather invasive and traumatic and substantially consist of a Rehfurs tube and gastroscopic instruments.

### Purposes of the invention

The main purpose of this invention is, in this respect, to provide a device as little invasive and traumatic as possible for sampling gastric juices from the gastric cavity;
another purpose of this invention is to achieve the previous purpose through a device that permits sampling significant quantities of gastric juices in order to test the samples for tumorous *markers* or antigenic or oncogenic substances;
yet another purpose of this invention is to achieve the previous purposes through a device that permits the application of known test methods without any particular complications of relevant implementation methods and furthermore without imposing particularly difficult and expensive adaptations;
another purpose of this invention is to achieve the previous purposes through a simple and functional device, which is easy to use and relatively inexpensive considering the results it is able to produce.

### Summary extract of the solution concept

These and other purposes still are all achieved with the endogastric capsule for sampling gastric juices according to this invention, comprising a removable absorbing medium (11) retained within a container (2) and communicating outside through at least one opening (10) as well as enclosed inside a dissolvable casing (3) and fastened to a string (4) for endogastric placement and relevant recovery.

### Finding the attached drawings

Further characteristics and advantages of the device according to this invention will appear more clearly evident from the detailed description that follows of one of its forms of implementation, preferred but not exclusive, represented by way of example only and not limited in the number two tables of drawings attached, in which:
figure 1 shows a view of an endogastric capsule according to this invention, fully assembled;
figure 2 shows the same structure partially split down into its relevant component parts;
figure 3 shows the same structure fully broken down into its component parts.

### Static description of the preferred example of realisation

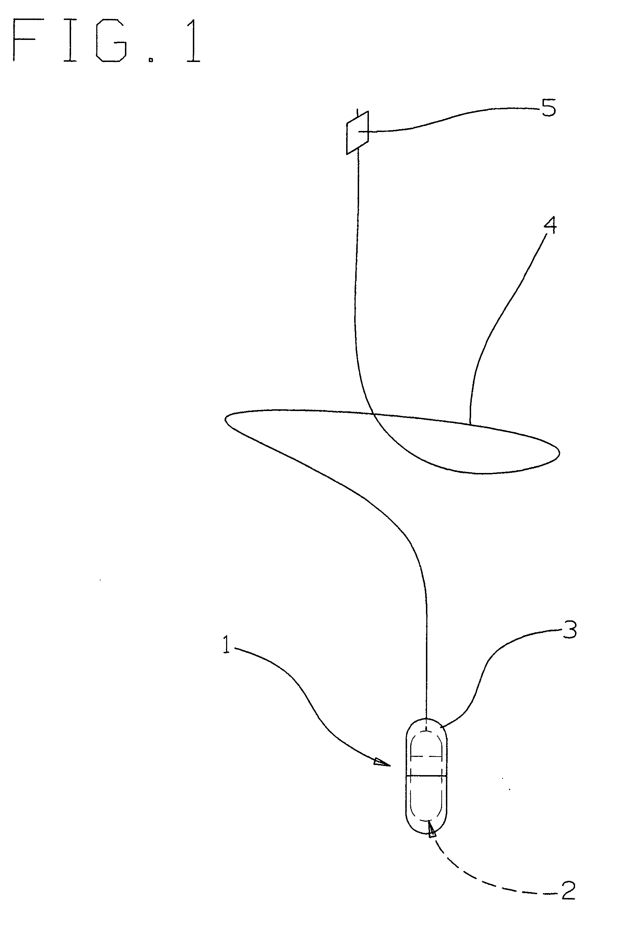

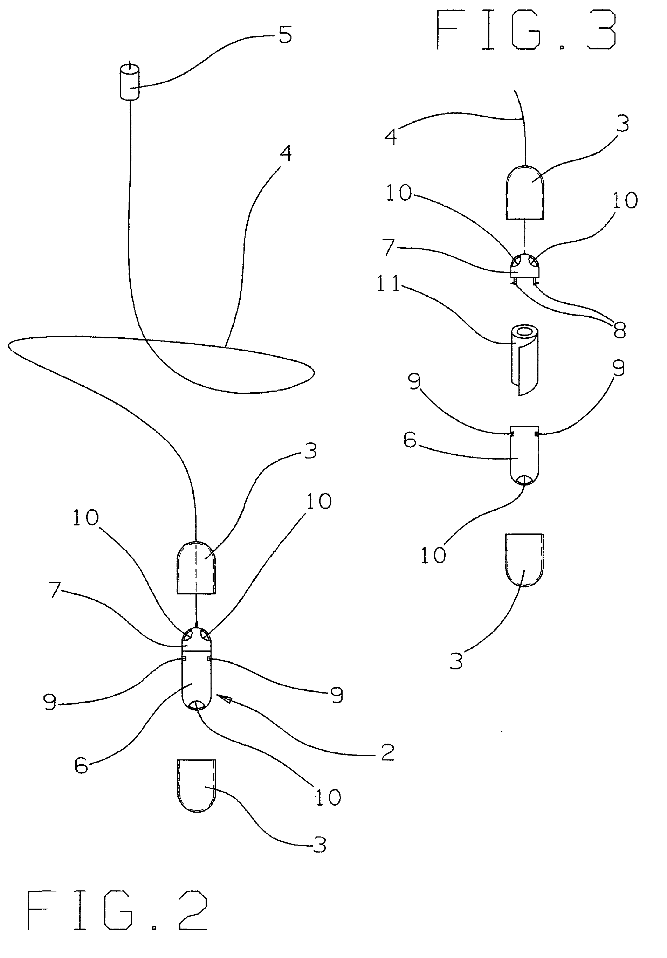

With reference to such figures and especially to figure 1, by 1 is indicated in its assembled entirety an endogastric capsule according to this invention, consisting (see figure 2) of a cylinder-shaped container with dome shaped bases, indicated in its entirety by 2 and preferably made of inert and harmless plastic material, preferably with dimensions 1.3 cm in length and 0.4 cm in diameter, enclosed inside a similarly shaped dissolvable casing 3, split into two symmetric halves reciprocally joinable in circumference by fitting a part of one into the other, as well as consisting of gelatine in the same way as the known storage capsules usually for powdery preparations to be taken through the mouth, being futher substantially sized like these, preferably 1.4 cm in length and 0.5 cm in diameter.

The cylinder-shaped container 2 is united to a string 4, preferably of the nylon type of any appropriate reduced section, preferably with a length variable between 45 and 50 cm, on the other end of which is fastened a grain-shaped body 5.

The cylinder-shaped container 2 consists of a case 6 and relevant lid 7 reciprocally unitable by means of elastic projections 8 contrasting and interacting with slots 9 made on the shell of the case 6.

Both the case 6 and the relevant lid 7 feature small holes 10 in their respective dome-shaped sections.
within the cylinder-shaped container 2 consisting of a case 6 and relevant lid 7 is a rolled-up sheet of absorbing paper, for absorbing the gastric juice samples illustrated below.

### Dynamic description of the preferred example of realisation

Having thus completed the static description of a preferred example of implementation of the device in accordance with this invention, we now go on to give a dynamic description, meaning relevant operation:
capsule 1, duly assembled into its above-mentioned component parts as illustrated in figure 1, can be swallowed by the patient just like any "wafer", if necessary accompanied by a sip of water, keeping however outside the mouth the other end of the string 4 to which it is tied, so that such string unwinds along the esophagus and makes it possible to introduce the capsule into the gastric cavity, the string being specially conceived of the appropriate length;
the external end of string 4 can be held by the patient using two fingers during the swallowing process, or else retained inside the mouth with the aid of the grain-shaped body 5, for the purpose fastenable to the dental arch, in the space between two teeth, so that during endogastric placement, the patient is substantially free and, though while not being able to perform any occupation, is definitely able to move in virtually complete freedom.

When the capsule 1 reaches the gastric cavity, the dissolvable casing 3 in gelatine, provided to make swallowing easier, dissolves in accordance with its nature, leaving the container 2 uncovered;
it must be noted how the dissolving of the casing 3 only occurs in the gastric cavity thus preventing pollution of the contents in the mouth and esophagus.

Through the small holes 10 of container 2, the gastric juices thus start to soak into the asorbing sheet 11, which in a time of about forty-five - sixty minutes becomes completely saturated;
at this point, the container 2 can be removed with a light and continuous traction on string 4 and this way, given its relevant dimensions and conformation, it can be easily removed without any substantial injury to the patient.

The absorbing paper 11 can then be freed by removing the lid 7 of case 6 and placed for about one hour in an appropriate quantity of physiological saline solution by means of a mixer, so that the gastric juice sample is mixed in such solution;
such solution can then be tested to determine the presence of tumorous *markers* or other antigens using the radioimmunological method or other known methods.

### Implementation alternatives

It is obvious that alternative forms of implementation, though always falling within the concept of solution aimed at the example of realisation shown above and claimed below, the device according to this invention can be implemented differently and realised, with technical and mechanical equivalents or supplementary measures, though always part of the same solution concept illustrated above and claimed below;
in particular, by way of example only and not obligatory, nor even more so, restrictively, container 2, besides having any dimension suitable for the purpose, can be shaped and structured in any way useful for placing and removing an absorbing medium within it, which, in turn, can be alternatively made of any appropriate material able to soak up in times and quantities suitable for the operation, for instance a sponge.

### Advantages of the invention

As appears evident from the previous detailed description of a preferred but not exclusive form of realisation and the mention also made above of alternative forms of implementation, the device according to this invention offers advantages corresponding to the preset purposes and others still:
it does in fact represent a simple, inexpensive and functional structure able to take samples of gastric juices without injuring the patient so as to permit successful monitoring of parameters that could relate to the onset of gastric tumours, i.e. strong tumorous markers such as for instance "CEA" and "CA19.9".

### Scope of requested protection

Having thus described the invention with reference to a preferred form of realisation, the desire is obvious to protect all further forms of implementation which are part of the same solution concept as applicable by an industry technician; this way it is intended, both in the preceding description as in the following claims, to protect all the forms of construction and the variations that fall within the spirit and purposes of the invention itself.

### KEY TO NUMBERS

1) complete capsule
2 ) complete cylinder-shaped container
3 ) dissolvable casing
4) string
5) grain-shaped body
6 ) case making up the cylinder-shaped container
7 ) lid featuring the cylinder-shaped container
8 ) elastic retention projections
9 ) relevant slots
10 ) small holes in cylinder-shaped container
11) absorbing paper

## Claims

1. Capsule **characterised by** the fact of comprising a removable absorbing medium (11) retained within a container (2) and communicating outside through at least one opening (10) as well as enclosed inside a dissolvable casing (3) and fastened to a string (4) for endogastric placement for sampling gastric juices and relevant recovery.

2. Capsule as claimed in claim 1 **characterised by** the fact that said absorbing medium consists of a rolled sheet of absorbing paper.

3. Capsule as claimed in claim 1 **characterised by** the fact that said container (2) consists of a cylinder-shaped structure (2) with dome-shaped bases consisting of a case (6) and relevant lid (7) fastened by means of elastic projections (8) contrasting and interacting with slots (9) made in the shell of said case (6).

4. Capsule as claimed in claims 1 and 3, **characterised by** the fact that said case (6) and said lid (7) feature small holes (10) on their respective dome-shaped portions.

5. Capsule as claimed in claim 1, **characterised by** the fact that said dissolvable casing (3) consists of a cylinder-shaped structure with dome-shaped bases split into two symmetric halves reciprocally joinable in circumference by fitting a part of one into the other.

6. Capsule as claimed in claim 1 **characterised by** the fact that said string (4) is fastened to said container (2) and the other end of said string is attached to grain-shaped body (5).

7. Capsule as claimed in claim 1 **characterised by** the fact that said absorbing medium consists of any material suitable for the purpose.

8. Capsule as claimed in claim 1 **characterised by** the fact that said container (2) is structured in any way suitable for the purpose.

9. Capsule as claimed in claim 1 **characterised by** the fact that said container (2) consists of any material suitable for the purpose.

10. Capsule as claimed in claim 1 **characterised by** the fact that said container (2) is of any size suitable for the purpose.

11. Capsule as claimed in claim 1, **characterised by** the fact that said small holes (10) feature in said container (2) in any number, size or position suitable for the purpose.

12. Capsule as claimed in claim 1, **characterised by** the fact that said dissolvable casing (3) is structured in any way suitable for the purpose.

13. Capsule as claimed in claim 1, **characterised by** the fact that said dissolvable casing (3) consists of any material suitable for the purpose.

14. Capsule as claimed in claim 1, **characterised by** the fact that said dissolvable casing (3) is of any size suitable for the purpose.

15. Capsule as claimed in claim 1, **characterised by** the fact that said string (4) is of any size suitable for the purpose.

16. Capsule as claimed in claims 1 and 6, **characterised by** the fact that said grain-shaped body (5) is sized and shaped in any way suitable for the purpose.
